# EUROPÄISCHE PATENTSCHRIFT

(11) **EP 1 023 079 B1**
(45) Veröffentlichungstag und Bekanntmachung des Hinweises auf die Patenterteilung: **29.08.2001**
(21) Anmeldenummer: 98946207.2
(22) Anmeldetag: 12.10.1998
(51) Int. Cl.: A61K 35/78

(54) **VERFAHREN ZUR HERSTELLUNG VON HEILPFLANZENEXTRAKTEN**
METHOD FOR PRODUCING MEDICINAL PLANT EXTRACTS
PROCEDE DE PRODUCTION D'EXTRAITS DE PLANTES MEDICINALES

(30) Priorität: 13.10.1997 EP 97117669
(43) Veröffentlichungstag der Anmeldung: 02.08.2000
(73) Patentinhaber: Max Zeller Söhne AG, 8590 Romanshorn (CH)
(72) Erfinder: STEINER, Rudolf, D-91056 Erlangen (DE); HAUK, Alexander, D-92242 Hirschau (DE); TRATZ, Werner, D-91052 Erlangen (DE)
(74) Vertreter: Ritscher, Thomas, Dr.
(86) Internationale Anmeldenummer: CH9800437
(87) Internationale Veröffentlichungsnummer: WO9918984

(56) Entgegenhaltungen:
- EP-A- 0 081 231
- EP-A- 0 391 504
- DE-A- 4 002 938

## Beschreibung

Die vorliegende Erfindung betrifft ein Verfahren zur Herstellung von phytochemisch wirksamen Extrakten aus einem Pflanzenmaterial, das neben lipophilen Wirkstoffen auch Anteile an polaren oder potentiell polaren Komponenten enthält, durch Extraktion mit einem selektiven Lösungsmittel, das die lipophilen Anteile besser löst, als die polaren Komponenten.

Die systematisch-wissenschaftliche Untersuchung der medizinischen Wirkung von Heilpflanzen ist allgemein Gegenstand der Phytotherapie, deren spezielle Aufgabe insbesondere die Verwendbarkeit und Gewinnung von aus Pflanzen gewonnen Präparaten zur therapeutischen Anwendung als Arzneimittel in der Human- und auch der Veterinärmedizin ist.

Trotz aller Fortschritte der Therapie mit halb- oder vollsynthetisch hergestellten Medikamenten hat die Phytotherapie einen festen Platz in der Behandlung von Krankheiten. Hierzu haben neuerdings verfügbare Methoden zur kritischen Beurteilung der therapeutischen Wirkungen von aus Pflanzen gewonnen Mitteln beigetragen, die es gestatten, eine über reine Placebo-Effekte hinausgehende Wirkung pharmakologisch zu sichern und zu bewerten. Ferner ist durch moderne analytische Methoden auch eine Standardisierung von direkt aus Pflanzen gewonnenen Produkten möglich, was in Anbetracht der insbesondere klimatisch bedingten Unterschiede von Wirkstoffart und Wirkstoffgehalt in Pflanzen aus verschiedenen Ernten für eine rationale Anwendung pflanzlicher Extrakte wesentlich ist.

Die hier verwendete Bezeichnung "phytochemisch wirksamer Extrakt" bezieht sich auf durch Extraktion aus Pflanzen gewonnene Produkte mit anerkannter therapeutischer Wirkung und definierter Qualität.

Hierbei werden als "Extrakte" sowohl primäre flüssige Extrakte als auch durch Lösungsmittelreduktion gewonnene "sekundäre" dickflüssige (spissum) sowie schliesslich die durch Lösungsmittelentfernung aus den flüssigen Extrakten erhältlichen festen Extrakte verstanden, die im typischen Fall mehrere Verbindungen, auch unterschiedlicher Art und Wirkung, enthalten.

Bei der Gewinnung von Extrakten sind zwei Parameter von Bedeutung: die Extraktausbeute, d.h. die aus einer gegebenen Menge Pflanzenmaterial gewonnene Menge an Extrakt, und die Extraktqualität, d.h. der Extrakt enthält die gewünschten (Wert)Stoffe und keine unerwünschten Komponenten.

Die Extraktqualität spielt insbesondere dann eine kritische Rolle, wenn das extrahierte Pflanzenmaterial Komponenten enthält, die störend oder gar toxisch sind, wie dies z.B. bei Petasites hybridus (Pestwurz) der Fall ist.

Therapeutische Verwendungen und Probleme der Gewinnung von Petasites-Extrakten sind in der deutschen Patentanmeldung DE 197 02 168 der Anmelderin ausführlich erläutert, auf welche Anmeldung hier durch Verweisung Bezug genommen wird. Darin sind insbesondere auch die aus DE-A-39 10 831 bzw. EP 0 391 504 und DE-A-41 41 749 bekannten Aspekte der kritischen Abtrennung der gewünschten Inhaltsstoffe ("Wertstoffe") von unerwünschten weil toxischen Pyrrolizidin-Alkaloiden ("Schadstoffe") einschliesslich der N-Oxide hiervon (nachfolgend gemeinsam kurz als PA bezeichnet) beschrieben.

Die Verwendung von selektiv wirkenden Extraktionsmitteln ist eine an sich übliche Methode für eine solche Trennung. Auf mehreren Gebieten der Verarbeitung und Behandlung von Pflanzenmaterial wird Kohlendioxid unter erhöhtem Druck als Extraktionsmittel verwendet, und es gibt eine umfangreiche Literatur über die selektive Extraktion von Tabak zur Gewinnung von Tabakaromastoffen (siehe z.B. EP 0 081231 bzw. DE-C2-31 48 335 und darin erwähnter Stand der Technik) sowie die Decoffeinierung von Kaffee und Tee (siehe z.B. Monographie von Stahl, E. et al., Verdichtete Gase zur Extraktion und Raffination, Springer Verlag 1987) mit Hilfe von Kohlendioxid als selektivem Extraktionsmittel, wobei auch eine Extraktion zur Gewinnung von Tabaköl in Gegenwart von Adsorptionsmittel beschrieben ist.

In der Monographie von Stahl et al, wird dabei ausgeführt (Seite 199), dass die Extraktion mit flüssigem (d.h. unterkritischem) Kohlendioxid wegen der im Vergleich zu überkritischem Kohlendioxid geringeren Selektivität nicht zweckmässig ist. Tatsächlich wird dann auch in Übereinstimmung mit der Monographie von Stahl et al. in dem aus EP 0' 391 504 bekannten Verfahren zur Gewinnung von Extrakten aus Petasites hybridus Kohlendioxid in überkritischem Zustand als selektives Lösungsmittel verwendet, um den PA-Anteil des Extraktes auf unter 0.1 ppm zu senken, was laut Patentschrift der Nachweisgrenze entsprechen soll. Ein solches Verfahren ist auch in DE 40 02 938 beschrieben, bei dem verdichtetes Kohlendioxid verwendet wird, womit ausweislich der Beispiele aber durchwegs auf überkritisches Kohlendioxid Bezug genommen wird.

Bei Versuchen der Nacharbeitung der Angaben von EP 0 391 504 konnten trotz verbesserter Analytik, die noch PA-Anteile von bis etwa 50 ppb (= 0.05 ppm) zu erfassen gestattet, unter den angegebenen Bedingungen die in dieser Schrift angegebenen niedrigen PA-Werte im unmittelbar gewonnen Extrakt jedoch nicht erzielt werden.

Im Zuge dieser Untersuchungen (Diplomarbeit Alexander Hauk, Herstellung von Pestwurzextrakten durch Extraktion mit hochverdichtetem CO₂, Lehrstuhl für Technische Chemie II, Friedrich-Alexander-Universität, Erlangen-Nürnberg) wurde jedoch gefunden, dass sich PA-Anteile des Extraktes von deutlich unter 0.1 ppm überraschenderweise dann erzielen lassen, wenn entgegen den Lehren des Standes der Technik Kohlendioxid gemäss der vorliegenden Erfindung in unterkritischem flüssigen Zustand als selektives Extraktionsmittel verwendet wird.

Gegenstand der Erfindung ist daher ein Verfahren zur Herstellung von phytochemisch wirksamen Extrakten aus Pflanzenmaterial, das wie Petasites hybridus neben lipophilen Wirkstoffen auch Anteile an polaren oder potentiell polaren Komponenten enthält, durch Extraktion mit einem selektiven Lösungsmittel, das die lipophilen Anteile besser löst, als die polaren Komponenten, und das dadurch gekennzeichnet ist, dass als Lösungsmittel Kohlendioxid bei unterkritischen Bedingungen verwendet wird.

Dadurch lässt sich der Aufwand zur Entfernung der PA-Anteile aus dem Extrakt signifikant verringern und die Wirtschaftlichkeit der Herstellung entsprechend verbessern.

Als polare oder potentiell polare Komponenten werden hier solche Anteile des Pflanzenmaterials verstanden, die ein signifikantes Dipolmoment haben, z.B. Komponenten mit basischen Gruppen, wie Alkaloide, die dem Molekül ein elektrisches Dipolmoment verleihen oder/und durch geeignete Behandlung polar bzw. stärker polar gemacht werden können. Folge der gegebenenfalls verstärkten Polarität ist ein entsprechend hydrophiler Charakter und dadurch eine bis zur Bedeutungslosigkeit verminderte Löslichkeit in flüssigem unterkritischem CO₂. Mit anderen Worten kann die Selektivität von flüssigem unterkritischem CO₂ für die Extraktion von lipophilen Komponenten um so mehr erhöht werden, als der polare Charakter der nicht im Extrakt gewünschten Komponenten verstärkt wird, und zwar überraschenderweise offenbar in höherem Masse, als dies bei überkritischem CO₂ der Fall ist.

Das besonders bevorzugte Pflanzenmaterial zur Verarbeitung nach dem erfindungsgemässen Verfahren ist ein solches von Petasites sp., insbesondere Petasites hybridus, das bekanntlich Anteile an Pyrrolizidin-Akaloidverbindungen (PA) enthält.

Allgemein wird das Kohlendioxid als Extraktionsmittel beim erfindungsgemässen Verfahren in flüssig-unterkritischem Zustande, d.h. allgemein bei einer Temperatur von < 31.06 °C, vorzugsweise ≤ 31 °C, insbesondere 0 - 30°C, verwendet. Bei der kritischen Temperatur von < 31.06 °C beträgt der kritische Druck 73.83 bar; allgemein liegt der Druck, bei dem Kohlendioxid noch im unterkritisch-flüssigen Zustand vorliegt, umso höher, je tiefer die Temperatur unterhalb der kritischen Temperatur liegt. Bei einer typischen Ausrührungsform des Verfahrens der Erfindung wird z.B. mit einem Druck von etwa 65 bar bei einer Temperatur von etwa 20°C und einer Extraktionsdauer von etwa 30 min gearbeitet. Bei einer Extraktionstemperatur von etwa 10°C kann der Druck entsprechend höher, z.B. bei 90 bar, liegen, wobei dann in der Regel für eine optimale Ausbeute auch langere Extraktionszeiten, z.B. 2 - 3 Stunden, erforderlich sind

Gemäss einer allgemein bevorzugten Ausfuhrungsform des erfindungsgemässen Verfahrens wird das extrahierte Pflanzenmaterial nach der Behandlung mit dem flüssigen Kohlendioxid von diesem getrennt und letzteres zur Gewinnung eines im wesentlichen von Wasser (einer ebenfalls polaren Komponente) und Alkaloiden freien Extraktes abgedampft, z.B. in einem Expansionsraum bei Temperaturen von über 31°C.

Bei der Durchführung des erfindungsgemässen Verfahrens kann es zur weiteren Verringerung des Anteils an unerwünschten Alkaloidverbindungen im Extrakt zweckmässig sein, das vom extrahierten Pflanzenmaterial abgetrennte aber Extrakt enthaltende flüssige Kohlendioxid mit einem Adsorptionsmittel zu behandeln, um allfällige restliche polare Anteile aus dem flüssigen Kohlendioxid zu entfernen Dies kann durch Rezyklieren während der Adsorption oder nachfolgend geschehen.

Als Adsorptionsmittel sind neben den typischen Adsorbentien, wie Siliciumdioxid in vorzugsweise kolloidaler Form, Aluminiumoxid, Aktivkohle, auch Ionenaustauscher zur Entfernung von Pyrrolizidin-Alkaloiden und/oder deren N-Oxiden geeignet, vorzugsweise Kationenaustauscher in H⁺-Form, z.B. ein solcher auf Basis eines sauren Polystyrolharzes, wie es technisch unter der Bezeichnung Lewatit® SC 104 (Bayer-Katalysator K 1221 der Firma Bayer AG erhältlich ist, oder ein makroretikularer Ionenaustauscher, wie er z.B. unter der Bezeichnung Amberlyst® von der Firma Rohm & Haas erhältlich ist. Die Wahl und Verwendung von Adsorptionsmitteln für ein gegebenes Material liegt im Rahmen des fachlichen Könnens und kann durch einige einfache Versuche optimiert werden.

Das Pflanzenmaterial wird vor der erfindungsgemässen Extraktion meist mechanisch, z B. durch Zerkleinerung in einer Mahlanlage, z.B. einer Mühle geeigneter Bauart oder/und einem Walzenstuhl, vorbehandelt, kann aber auch chemisch oder mechanisch/chemisch vorbehandelt werden, z.B. durch Behandlung mit Säuren, Basen oder Enzymen, mit oder ohne gleichzeitige mechanische Einwirkungen. Für kommerziellen Betrieb ist oft eine Vorbehandlung des Pflanzenmaterials mit anorganischer oder organischer Säure, z.B. durch Besprühen mit verdünnter, z.B. 0,1 N wässriger Schwefelsäure, zweckmässig. Es wird angenommen, dass die basischen Pyrrolizidin-Alkaloide durch Säure in eine ionische Form überführt werden, wodurch ihre Polaritat erhöht und die Löslichkeit in dem als Extraktionslösungsmittel verwendeten unterkritisch-flüssigen CO₂ vermindert wird, der PA-Anteil somit vermehrt im Extraktionsrückstand verbleibt

Gegenstand der Erfindung ist ferner ein phytotherapeutisches Mittel, das mindestens teilweise aus Extrakt von Petasites hybridus besteht oder daraus gewonnen ist, welcher Extrakt nach dem erfindungsgemässen Verfahren erhalten ist, insbesondere ein Mittel, dessen Pyrrolizidin-Anteil im Extrakt (einschliesslich der N-Oxide) unter etwa 75 ppb (0.075 ppm) und typisch im Bereich von etwa 50 ppb oder weniger liegt.

Gegenstand der Erfindung ist schliesslich auch die Anwendung des erfindungsgemässen Verfahrens zur Extraktion von Pflanzenmaterial, das wie Petasites hybridus phytochemisch wirksame lipophile Anteile und unerwünschte polare oder potentiell polare Komponenten enthält.

Die chemische Zusammensetzung und die Strukturen der Komponenten der bevorzugten Extrakte aus Petasites hybridus sind für die Petasin-Varietat insbesondere aus Chimia 48 (1994) S. 564- 569 bekannt (siehe dort insbesondere die Formeln 1 - 4 und Tabellen 2 und 3), auf die hier durch Verweisung Bezug genommen wird. Neben Petasin finden sich in den Extrakten meist eine Reihe petasinähnlicher Stoffe, wie Isopetasin, Neopetasin, Desoxyneopetasol, Desoxyiso- und -neopetasol (einschliesslich der 13-substituierten Derivate hiervon), Methylcrotonylpetasol und -isopetasol, Methacryloyl-petasol und-isopetasol, Isobutyryl-neopetasol sowie Methylthioacryloyl-petasol, -neopetasol und-isopetasol.

Für die Furanopetasin-Varietät von Petasites hybridus sind die Strukturen der wichtigsten Komponenten (frisch bzw. gefriergetrocknet und luftgetrocknet) aus einer Dissertation (Siegenthaler; Untersuchungen zur Struktur und Analytik der Inhaltsstoffe von Petasites albus und Petasites hybridus (Furanopetasin-Varietät); Bern 1995), bekannt. Es handelt sich dabei insbesondere um die Verbindungsgruppen der Furanoeremophilane und-Furanoeremophilanlactone (s. Tabellen auf Seiten 198 und 199 der Dissertation Siegenthaler).

Zur Standardisierung erfindungsgemäss verwendeter Extrakte kann zweckmässig auf den Petasin/Isopetasingehalt (einschliesslich der entsprechenden Derivate, wie S-Petasin) bzw. auf den Furanoeremophilan-Gehalt (einschliesslich der entsprechenden Derivate) Bezug genommen werden.

Der gewonnen Extrakt kann mit den üblichen Zusatz- und Hilfsstoffen zu den an sich bekannten festen oder flüssigen Verabreichungsformen verarbeitet und in an sich bekannter Weise therapeutisch verwendet werden. In diesem Zusammenhang wurde gefunden, dass sich der gewonnene Petasites-Extrakt durch Zusatz von pharmakologisch verträglichen, wasserloslichen Polymeren, wie insbesondere Polyäthylenglykolen, selbst wasserlöslich machen lässt und eine solche wasserlösliche Zubereitung eine bevorzugte Ausführungsform eines erfindungsgemässen Petasites-Extraktes ist Hierzu sind insbesondere pharmakologisch zulässige Polyethylenglykole mit Zahlenmittelmolekulargewichten im Bereich von unter etwa 2000 und ähnliche synthetische, halbsynthetische oder natürliche wasserlösliche Polymere geeignet.

Zur Durchführung des erfindungsgemässen Verfahrens sind grundsätzlich diejenigen Vorrichtungen und Anlagen geeignet, die auch für die Extraktion mit überkritischem Kohlendioxid verwendet werden können; allgemein erforderlich ist, dass die Anlagen den speziell gewählten unterkritischen Bedingungen entsprechen müssen, was aber eine für Fachleute ohne weiteres erfüllbare Anweisung darstellt.

Eine zur Durchführung des neuen Verfahrens bevorzugte erfindungsgemässe Vorrichtung besitzt mindestens einen druckfesten Extraktionsbehälter, in den flüssiges Kohlendioxid bei unterkritischen Bedingungen eingeführt und von dem ein Strom von Extrakt enthaltendem flüssigen Kohlendioxid abgezogen werden kann, und ist gekennzeichnet durch mindestens eine Zone zur Behandlung des extrakthaltigen flüssigen Kohlendioxids mit Adsorptionsmittel, wobei die Zone im Extraktionsbehälter liegt oder/und diesem in einem separaten druckfesten Behälter nachgeschaltet ist.

Die Erfindung wird nachfolgend anhand der Zeichnung und anhand von Beispielen erläutert, ohne dass dies eine Beschränkung darstellt.

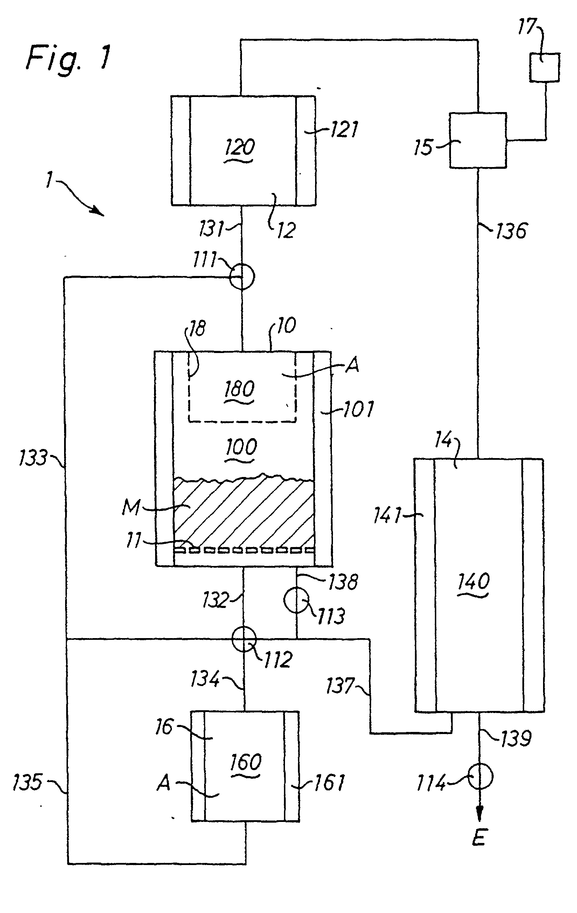

In der einzigen Figur 1 ist das Schema einer Vorrichtung 1 zur Durchführung des Verfahrens dargestellt. Die Vorrichtung 1 umfasst mindestens einen Extraktionsbehälter 10 (nachfolgend kurz Extraktor genannt), der ausreichend druckbeständig zum Betrieb mit flüssigem Kohlendioxid bei unterkritischen Bedingungen geeignet ist. Allgemein sollten der Extraktor und alle den Extrakt führenden und aufnehmenden Teile einer zur Durchführung des erfindungsgemässen Verfahrens verwendeten Anlage einen Betriebsdruck von mindestens 50 bar und vorzugsweise etwa 100 bar zulassen.

Der Extraktor 10 hat einen Innenraum 100 zur Aufnahme und Rückhaltung von zerkleinertem Pflanzenmaterial M und einen Doppelmantel 101 zur Temperierung des Innenraums 100. Ferner ist der Extraktor 10 mit Mess- und Regeleinrichtungen versehen, um die Einhaltung einer gegebenen Extraktionstemperatur bei unterkritischen Bedingungen des flüssigen CO₂ zu gewährleisten. Im Extraktor 10 ist ein für flüssiges Kohlendioxid durchlässiger Boden 11 angebracht, der das in den Extraktionsraum 100 eingeführte Pflanzenmaterial im Extraktor 10 zurückhält.

Der Extraktor 10 ist durch die Leitung 131 über das Ventil 111 mit einer Quelle 12 für flüssiges Kohlendioxid verbunden. Die Quelle 12 ist im typischen Fall ein druckfester (z.B. bis 150 bar) Behälter, der mit einem Doppelmantel 121 zur Temperierung und mit (nicht dargestellten) Temperatur- und Druckmesseinrichtungen versehen ist. Die Quelle 12 ist ferner mit einer Anlage 15 zur Verflüssigung von gasförmigem Kohlendioxid verbunden, die über die Leitung 136 mit rezykliertem gasförmigem CO₂ gespeist wird. Zur Erstspeisung mit bzw. Ergänzung von CO₂ kann die Anlage 15 mit einem Druckbehälter 17 verbunden werden, der gasförmiges CO₂ enthält.

In der Leitung 131 ist ein Umschaltventil 111 angeordnet, um die Zufuhr von Kohlendioxid von der Quelle 12 zu drosseln oder abzuschalten, wenn der den Extraktor 10 durch die Leitung 132 verlassende Strom aus flüssigem Kohlendioxid mit darin enthaltenem Extraktmaterial (Extraktionsstrom) über das Steuerventil 112 und die Leitung 133 in den Extraktor 10 rezirkuliert wird.

Wenn der Extraktionsstrom gemäss einer bevorzugten Ausführungsform des erfindungsgemässen Verfahrens mit festem Adsorptionsmittel A behandelt werden soll, kann dies in einem separaten Behälter 16 geschehen, der das Absorptionsmittel in der Zone 160 enthält und durch die Leitung 134 mit dem Extraktionsstrom gespeist wird, indem das Ventil 112 in die zur Beschickung des Behälters 16 durch die Leitung 134 mit dem gesamten Extraktionsstrom oder einem Teil desselben erforderliche Stellung gebracht worden ist. Der den Behälter 16 verlassende Extraktionsstrom gelangt dann durch die Leitung 135 in die Leitung 133 und wieder in den Extraktor 10.

Alternativ oder komplementär kann Adsorptionsmittel A auch in einer Zone 180 vorgelegt werden, die im Extraktor 10 liegt und z.B. von einem Einsatz 18 gebildet wird, der im oberen Teil des Extraktors 10 liegt und für das flüssige Kohlendioxid durchlässig ist, das Adsorptionsmittel aber zurückhält.

Beim Erreichen der gewünschten Extraktkonzentration kann der Extraktionsstrom entweder durch die Leitung 138 und das Ventil 113 oder die Leitung 132 und das entsprechend umgestellte Ventil 112 in die Leitung 137 und in den Expansionsbehälter 14 überführt werden, in dessen Innenraum 140 das CO₂ verdampft wird und dadurch der über die Leitung 139 und das Ventil 114 abziehbare Extrakt E gewonnen wird. Der Expansionsbehälter 14 ist mit einem Heizmantel 141 versehen, um die zur Verdampfung des CO₂ erforderliche Wärme zuzuführen. Das gasförmige CO₂ gelangt dann über die Leitung 136 in die Verflüssigungsanlage 15 und von dort in den Vorratsbehälter 12

Die unerwünschten polaren Anteile, d.h. im vorliegenden Fall hauptsächlich Pyrrolizidin-Alkaloide und Wasser, verbleiben im Extraktionsrückstand, der in üblicher Weise durch Verbrennen oder durch Verwendung als Dünger entsorgt werden kann.

Die Einzelheiten der Steuerung und Überwachung des Extraktionsvorgangs in der Anlage 1 und der Energiekonservierung beim Verdampfen und Verflüssigen des Kohlendioxids durch entsprechende Wärmetauscher sind in Fig. 1 nicht dargestellt, da sie im Bereich des Fachwissens liegen.

Die nachfolgenden Ausführungen dienen der weiteren Erläuterung der Erfindung, ohne diese zu beschränken.

### BEISPIELE

Die Beispiele wurden in einer Anlage durchgeführt, die im Prinzip dem in Figur 1 dargestellten Schema entsprach. Die der Quelle 12 für flüssiges Kohlendioxid vorgeschaltete Verflüssigungsanlage 15 wurde mit gasförmigem handelsüblichem Kohlendioxid, z.B. aus üblichen Bomben 17, gespeist. Die Quelle selbst war für einen maximalen Betriebsdruck von etwa 150 bar ausgelegt und mit einem äusseren Kühlmantel 121 und einer (in Fig. 1 nicht dargestellten) inneren Kühlschlange versehen. Als Kühlmittel diente ein Glykol-Wassergemisch.

Der Extraktor 10 war ein Hochdruck-Autoklav der Firma Nowa Swiss (Effretikon, Schweiz) aus rostfreiem Stahl mit einem zylindrischen Innenraum und einem Volumen von 400 ml. Der CO₂-Strom wurde mit einem Durchflussmessgerät (Nicro-Motion) bestimmt. Als Förderpumpen wurden Membranpumpen (Nominalleistung 10.9 Liter/Stunde) - verwendet. Einstellung und Regelung des Arbeitsdrucks im Extraktor 10 erfolgte über ein Dosierventil mit Schrittmotor. Für die Druckmessung wurde ein Präzisionsmanometer (Manom AG, Hitzkirch) verwendet. Der Betrieb der Anlage konnte manuell oder automatisch gesteuert werden.

Das für die Versuche als Ausgangsmaterial verwendete Pflanzenmaterial bestand aus Wurzelstock (Rhizom) von Petasites hybridus, der bis zu einer mittleren Korngrösse von etwa 500µm zerkleinert worden war.

Als Charge wurden 160 g Pflanzenmaterial (Feuchtigkeitsgehalt 6 bzw. 9 Gew.%) in den Extraktor eingefüllt und 30 Minuten mit flüssigem Kohlendioxid bei 65 bar und 20°C mit einem CO₂-Durchsatz von 25 kg CO₂/kg Pflanzenmaterial·Stunde extrahiert. Der Schadstoffgehalt des Pflanzenmaterials (Pyrrolizidin-Alkaloide und N-Oxide) betrug 40 ppm (40'000 ppb).

### Beispiel 1

Bei diesem Versuch wurde das Ausgangsmaterial als solches verwendet, d.h. weder vorbehandelt noch der Extrakt mit einem Adsorbens nachgereinigt. Es wurden 5,03 g eines klaren hellgelben, viskosen Petasites-Extraktes mit einem Gehalt an Pyrrolizidin-Alkaloiden von 0.874 ppm (=874 ppb) erhalten.

### Beispiel 2

Bei diesem Versuch wurde das Ausgangsmaterial in einem Vorbehandlungsschritt mit 6 g 0,1 N-H₂SO₄ besprüht und etwa 15 Stunden zur Einwirkung der Säure stehengelassen. Dann wurde es in den Autoklaven eingefüllt und wie beschrieben mit flüssigem CO₂ extrahiert. Es wurden 4,64 g eines klaren, hellgelben, viskosen Petasites-Extraktes mit einem PA-Gehalt von 566 ppb erhalten.

### Beispiel 3

Bei diesem Versuch wurde das Ausgangsmaterial nicht vorbehandelt, aber es wurden zusätzlich zu den 160 g zerkleinerten Pestwurz-Rhizomen noch 70 g des stark sauren Kationentauscherharzes Bayer Katalysatorkomponente K 1221 (früher Lewatit SC 104) in den Adsorbensbehälter 18 geschichtet. Bei der Extraktion wurden 5,00 g eines ebenfalls klaren, hellgelben, viskosen Petasites-Extrakts mit einem PA-Gehalt von 133 ppb erhalten.

### Beispiel 4

Beim diesem Versuch wurde das Ausgangsmaterial wie in Beispiel 2 mit 6 g 0,1 N-H₂SO₄ besprüht und anschliessend etwa 15 h zur Einwirkung der Säure stehengelassen. Wie in Beispiel 3 wurde die Adsorbens-Zone 180 des Extraktors 10 mit 70 g Adsorberharz beschickt; dann wurde mit flüssigem CO₂ extrahiert. Es wurden 4,53 g Patasites-Extrakt mit einem PA-Gehalt von 37 ppb (=0.037 ppm) erhalten. Dieser Wert liegt im Bereich der Nachweisgrenze der angewendeten Analysenmethodik (GC-Analyse).

Allgemein werden die qualitativ hochwertigsten Extrakte mit PA-Gehalten unter 0.075 ppm (75 ppb) erhalten, wenn die zerkleinerten Pestwurz-Rhizome durch Säureeinwirkung vorbehandelt, mit unterkritischem flüssigem CO₂ extrahiert und der rezirkulierte Extraktstrom mit Adsorbens behandelt wird

### VERGLEICHSBEISPIELE

Um die gegenüber dem Stand der Technik verbesserte Wirkungsweise der erfindungsgemässen Extraktion mit flüssigem unterkritischem CO₂ zu belegen, wurden Vergleichsversuche unter grundsätzlich gleichen Bedingungen wie in den obigen erfindungsgemässen Beispielen durchgeführt. Unterschiedlich waren lediglich die Druck-Temperatur-Bedingungen, die mit 250 bar und 45°C über dem kritischen Punkt des CO₂ (74 bar /31°C) lagen und damit dem Stand der Technik entsprachen.

### Vergleichsbeispiel 5 (Vergleich mit Beispiel 1)

Bei diesem Versuch wurde das Ausgangsmaterial wie in Beispiel 1 nicht vorbehandelt und auch nicht adsorptiv nachgereinigt. Aus 160 g zerkleinerten Pestwurz-Rhizomen wurden 6,64 g eines milchig-gelben Petasites-Extraktes mit einem Wassergehalt von ca. 20% und einem PA-Gehalt von 5.680 ppm (=5680 ppb) gewonnen.

Dieser Vergleich zeigt, dass die erfindungsgemässe Extraktion mit unterkritischem flüssigem CO₂ bereits ohne Vor- oder Nachbehandlung entscheidende Vorteile gegenüber dem Stand der Technik hat: zwar ist die Extraktausbeute bei der Extraktion mit überkritischem CO₂ höher, aber der dabei erhaltene Extrakt enthält erhebliche Mengen Wasser, ist deshalb milchig gefärbt und müsste nachträglich in eine wässrige und eine organische Phase getrennt werden, was einen zusätzlichen Aufwand erfordert.

Selbst nach Abtrennung der wässrigen Phase enthält die Wertstoffextraktion noch 3920 ppb PA. Bei der Extraktion mit flüssigem unterkritischem CO₂ wird hingegen nur eine organische Phase erhalten, die praktisch wasserfrei ist, weil das flüssige CO₂ das im Ausgangsmaterial enthaltene Wasser, das ein polarer Anteil des Pflanzenmaterials ist, nicht mitextrahiert. Der erfindungsgemäss erhaltene Extrakt enthält mit nur 874 ppb PA einen signifikant niedrigeren Schadstoffgehalt als das Produkt aus der Extraktion mit überkritischem CO₂.

### Vergleichsbeispiel 6 (Vergleich mit Beispiel 3)

Bei diesem Versuch wurde das Ausgangsmaterial wie in Beispiel 3 nicht vorbehandelt, der Adsorbensraum 180 aber ebenfalls mit 70 g Adsorberharz gefüllt. Es wurden 5,36 g eines klaren, aber dunkelgelben Petasites-Extrakts mit einem PA-Gehalt von 0.9 ppm (= 900 ppb) erhalten.

Auch dieser Vergleich zeigt deutlich die Vorteile der erfindungsgemässen Extraktion gegenüber der bekannten Extraktion mit überkritischem flüssigem Kohlendioxid. Zwar werden in beiden Fällen klare , d.h. praktisch wasserfreie Extrakte erhalten. Dies ist bei Extraktion mit überkritischem CO₂ gemäss Stand der Technik vermutlich im wesentlichen dadurch bedingt, dass das Wasser durch das Adsorbens aufgenommen wird. Beim erfindungsgemässen Verfahren ist die Entfernung von Wasser hingegen dem polaren Charakter von Wasser und der diesbezüglich negativen Selektivität von flüssigem unterkritischem CO₂ zuzuschreiben. Der erfindungsgemäss erhaltene Extrakt ist aber ausserdem deutlich heller und enthält vor allem signifikant weniger PA. nämlich nur 133 ppb gegenüber 900 ppb bei Verwendung von überkritischem CO₂ unter sonst gleichen Bedingungen.

Die Ergebnisse der obigen Beispiele sind in der nachfolgenden Tabelle zusammengestellt.

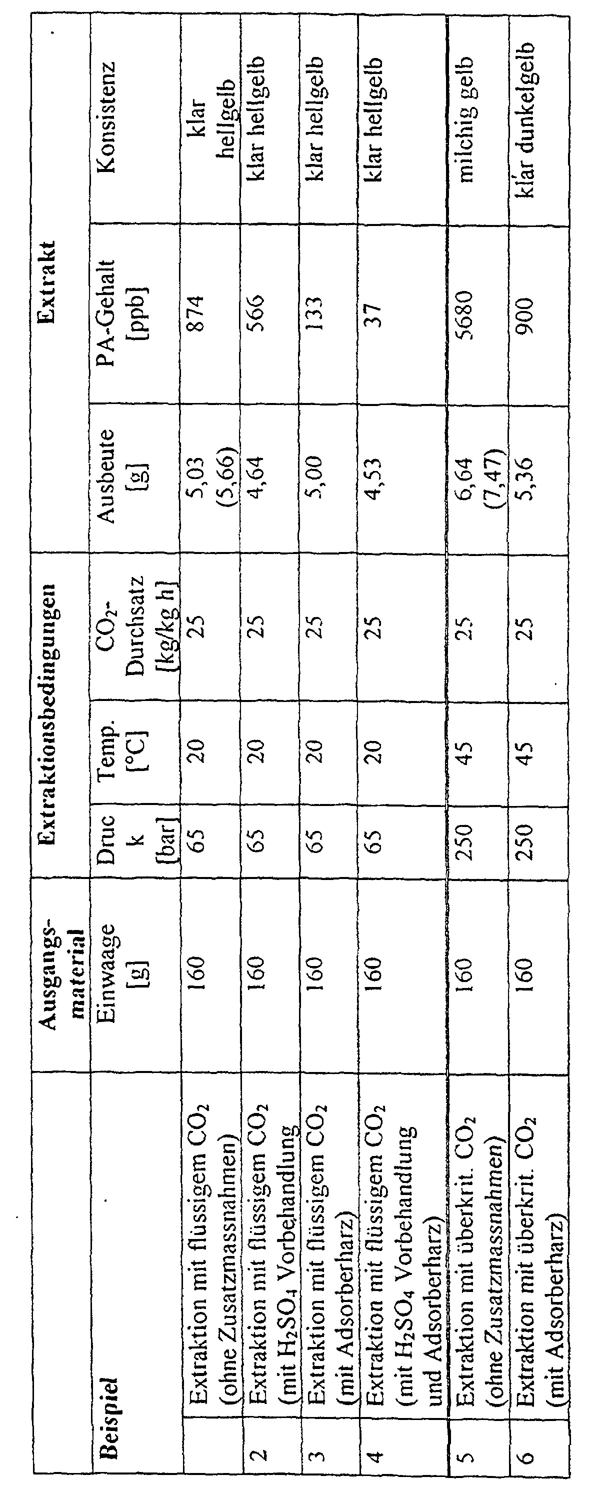

### Beispiel 7

Zur Durchführung des erfindungsgemässen Verfahrens in einer kommerziellen 3 x 500-Liter Extraktionsanlage wurde das Pflanzenmaterial (Blatt- oder/und Wurzelmaterial) auf einer Alpine-Universalmühle zerkleinert. Als Mahlwerkzeug diente ein Hammerschlagwerk mit 0,8 mm Riffelsieb; die Mahltemepratur betrug etwa minus 10°C. Der Wassergehalt des Mahlguts betrug ca. 9 %. Das Mahlgut wurde mit 0,1 N wässriger Schwefelsäure besprüht und dadurch auf einen Wassergehalt von etwa 15 Gew.% eingestellt. Das Gut wurde homogenisiert und mindestens über Nacht, vorzugsweise 2 - 3 Tage, zur Einwirkung der Säure stehen gelassen. Die Säure wurde vom Extraktionsgut vollständig aufgenommen so dass auch nach der Säurebehandlung ein rieselfähiges Material vorliegt.

Die Extraktion mit Chargen von je 50 kg Blattmaterial in den beiden oberen Extraktorsegmenten und 80 kg Ionenaustauscherharz (Bayer Katalysator K1221; zur Reinigung bzw. Regeneration mit Ethanol bis zu einer Adsorption von unter 0,2 extrahiert) im untersten Extraktorsegment bzw. je 100 kg Wurzelmaterial in den beiden oberen Extraktorsegmenten und 80 kg Ionenaustauscherharz im untersten Extraktorsegment wurde jeweils während 30 Minuten bis 2 Stunden mit einem CO₂-Durchfluss von ca 1'250 kg/Stunde bei Extraktionstemperaturen von etwa 10°C, 20°C und 30°C, jeweils bei unterkritischen Drücken von 90 bar, 70 bar und 60 bar, extrahiert. Die Abscheidung des Extraktes erfolgte jeweils durch Erhöhung von Druck und Temperatur und Verdampfen des CO₂, das nach der Abtrennung des Extraktes wieder verflüssigt und rezirkuliert wurde. Auf diese Weise wurden jeweils Extraktausbeuten von 1,4 - 5 Gew.%, bezogen auf das Pflanzenmaterial, mit PA-Anteilen von ≤ 0.075 ppm erhalten.

### Beispiel 8

Dieses Beispiel erläutert die Herstellung einer wasserlöslichen und tablettierbaren Zubereitung aus Petasin-Extrakt.

300 Gewichtsteile des nach der Arbeitsweise von Beispiel 4 erhaltenen Extraktes wurden mit 300 Gewichtsteilen Aerosil® 200V (pyrogenes Siliciumdioxid der Firma Degussa) wurden mit 300 Gewichtsteilen mikrokristalliner Cellulose in einem Stephan-Mischer vorgelegt und gemischt.

Dann wurden 240 Gewichtsteile Lutrol® F68 (Netzmittel; Polyethlenglykol der BASF) und 360 Gewichtsteile Lutrol® F127 (Polyäthylenglykol der BASF) bei ca. 75°C geschmolzen, portionenweise zugegeben und mit dem Rührwerk eingearbeitet. Das resultierende weisse Granulat wurde nach dem Sieben in einer üblichen Tablettierpresse zu Tabletten von 500 mg verarbeitet, deren Petasin-Extrakt unter physiologischen Bedingungen wasserlöslich ist.

Für Fachleute ergeben sich aus der obigen Beschreibung verschiedene Variationen des beschriebenen neuen Verfahrens, die jedoch im Rahmen des Schutzumfangs der nachfolgenden Ansprüche liegen. So kann z.B. die oben beschriebene chargenweise Extraktion kontinuierlich durchgeführt werden, z.B. indem mehrere Extraktoren parallel geschaltet und je für sich chargenweise betrieben werden, aber insgesamt ein kontinuierlicher Produktstrom entsteht. Alternativ können Hochdruckextraktoren für kontinuierlichen Betrieb und vorzugsweise im Gegenstrom erfolgenden Betrieb verwendet werden, indem das am stärksten extrahierte Pflanzenmaterial mit unbeladenem flüssigen unterkritischen CO₂ und das frische Pflanzenmaterial mit dem bereits extrakthaltigen CO₂ behandelt wird. Anstelle von Petasites hybridus können andere bekannte Heilmittelpflanzen erfindungsgemäss extrahiert werden Weitere Anpassungen an gegebene spezielle Verhältnisse sowie die Weiterverarbeitung der erhaltenen Extrakte liegen im Bereich des Fachmännischen und bedürfen keiner Erläuterung.

## Patentansprüche

1. Verfahren zur Herstellung von phytochemisch wirksamen Extrakten aus einem Pflanzenmaterial von Petasites sp., das neben lipophilen Wirkstoffen auch Anteile an polaren oder potentiell polaren Komponenten enthält, durch Extraktion mit einem Lösungsmittel, das die lipophilen Anteile besser löst, als die polaren Komponenten, **dadurch gekennzeichnet**, dass als Lösungsmittel flüssiges Kohlendioxid bei unterkritischen Temperatur bedingungen (≤ 31°C) verwendet wird.

2. Verfahren nach Anspruch 1, **dadurch gekennzeichnet**, dass das Pflanzenmaterial als polare Komponente(n) Wasser und/oder mindestens ein Alkaloid enthält, das polar ist oder durch sauren oder alkalischen Aufschluss polar gemacht werden kann.

3. Verfahren nach Anspruch 1 oder 2, **dadurch gekennzeichnet**, dass das Pflanzenmaterial von Petasites sp., insbesondere Petasites hybridus, Anteile an Pyrrolizidin-Verbindungen und/oder deren N-Oxide enthält.

4. Verfahren nach einem der Ansprüche i - 3, **dadurch gekennzeichnet**, dass flüssiges Kohlendioxid bei einer Temperatur von 0 - 30°C, als Extraktionsmittel verwendet wird.

5. Verfahren nach einem der Ansprüche 1 - 4, **dadurch gekennzeichnet**, dass das Pflanzenmaterial nach der Behandlung mit dem flüssigen Kohlendioxid von diesem getrennt und letzteres zur Gewinnung eines im wesentlichen von Wasser und Alkaloiden freien Extraktes abgedampft wird, z.B. in einem Autoklav bei Temperaturen von > 31°C.

6. Verfahren nach Anspruch 5, **dadurch gekennzeichnet**, dass das flüssige Kohlendioxid mit darin gelösten Anteilen des Pflanzenmaterials während oder nach der Extraktion mit einem Adsorptionsmittel behandelt wird, um restliche Anteile polarer Komponenten des Extrakts zu entfernen.

7. Verfahren nach einem der Ansprüche 1 - 6, **dadurch gekennzeichnet**, dass das Pflanzenmaterial mechamisch, z.B. durch Zerkleinerung, und/oder chemisch, z.B. durch Behandlung mit Säuren, Basen, Enzymen oder Ionenaustauschern, vorbehandelt wird.

## Claims

1. A method of producing a phytochemically effective extract from a plant material of Petasites sp. containing, in addition to lipophilic active ingredients, polar or potentially polar components by extraction with a solvent that dissolves the lipophilic active ingredients better than the polar components, **characterized in that** liquid carbon dioxide at sub-critical temperature conditions (≤31°C) is used as solvent.

2. The method of claim 1 **characterized in that** the plant material contains water as said polar component(s) and/or at least one alkaloid that is polar or can be made polar by acid or alkaline digestion.

3. The method of claim 1 or 2 **characterized in that** said plant material of Petasites sp, and notably Petasites of hybridus, contains pyrrolizidine compounds and/or N-oxides thereof a constituents.

4. The method of any of claims 1 - 3 **characterized in that** liquid carbon dioxide at a temperature of from 0 to 30°C is used as extraction agent.

5. The method of any of claims 1 - 4 **characterized in that** the plant material is separated from said carbon dioxide after treatment therewith, and that the latter is evaporated to yield an extract which is essentially from water and alkaloids, e.g. in an autoclave at temperatures of > 31°C.

6. The method of claim 5 **characterized in that** said liquid carbon dioxide with the components of the plant material dissolved therein is treated with and adsorptive agent during or after the extraction to remove residual portions of polar components from said extract.

7. The method of any of claims 1-6 **characterized in that** said plant material is pretreated mechanically, e.g. by comminution, and/or chemically, e.g. by treatment with acids, bases, enzymes or ion exchangers.

## Revendications

1. Procédé de production d'extraits à efficacité phytochimique à partir d'un matériau végétal de *Petasites* sp.; contenant à côté de substances actives lipophiles également des parts de composants polaires ou potentiellement polaires, par extraction avec un solvant, permettant une meilleure dissolution des parts lipophiles que les composants polaires, **caractérisé en ce qu**'on utilise comme solvant du dioxyde de carbone liquide en présence de conditions de température sous-critiques (≤ 31°C).

2. Procédé selon la revendication 1, **caractérisé en ce que** le matériau végétal contient comme composant(s) polaire(s) de l'eau et/ou au moins un alcaloïde polaire ou pouvant être rendu polaire par dissolution acide ou alcaline.

3. Procédé selon les revendications 1 ou 2, **caractérisé en ce que** le matériau végétal (de *Petasites* sp., en particulier de *Petasites hybridus*), contient des parts de composés pyrrolizidine et/ou des N-oxydes correspondants.

4. Procédé selon l'une des revendications 1 à 3, **caractérisé en ce qu**'on utilise du dioxyde de carbone liquide en présence d'une température comprise entre 0 et 30°C comme moyen d'extraction.

5. Procédé selon l'une des revendications 1 à 4, **caractérisé en ce qu**'après le traitement avec le dioxyde de carbone liquide, le matériau végétal est séparé de celui-ci et celui-ci est évaporé en vue de produire un extrait essentiellement exempt d'eau et d'alcaloïdes, par exemple dans un autoclave, en présence de températures supérieures à 31°C.

6. Procédé selon la revendication 5, **caractérisé en ce que** le dioxyde de carbone liquide avec les parts du matériau végétal qui y sont dissoutes est traité avec un adsorbant, au cours de l'extraction ou après celle-ci, en vue d'éliminer les parts résiduelles des composants polaires de l'extrait.

7. Procédé selon l'une des revendications 1 à 6, **caractérisé en ce que** le matériau végétal est soumis à un prétraitement mécanique, par exemple par fragmentation, et/ou chimique, par exemple par un traitement avec des acides, des bases, des enzymes ou des échangeurs d'ions.
